# EUROPEAN PATENT APPLICATION

(11) **EP 1 150 237 A2**
(43) Date of publication of application: **31.10.2001**
(21) Application number: 01303825.2
(22) Date of filing: 26.04.2001
(51) Int. Cl.: G06F 19/00

(54) **Wireless data communication by high frequency sound**

(30) Priority: 27.04.2000 US 560411
(71) Applicant: GE Marquette Medical Systems, Inc., Milwaukee, Wisconsin 53223 (US)
(72) Inventor: Reynolds, Charles A., West Haven, Connecticut 06516 (US)
(74) Representative: Pedder, James Cuthbert

(57) **Abstract**

A wireless data communication system (10) includes a transmit circuit (26) configured to receive a data signal and to generate a high frequency sound signal based on the received data signal and a receive circuit (30) spaced a distance from the transmit circuit (26) configured to receive the high frequency sound signal and to remove the data signal from the high frequency sound signal.

## Description

The invention relates generally to the field of data communication systems. More particularly, the invention relates to wireless data communication systems. More particularly still, the invention relates to wireless data communication systems in the medical diagnostics field.

Medical diagnostic systems are used extensively in modern health care facilities. Such systems are invaluable tools for identifying, diagnosing and treating physical conditions. One such medical diagnostic system is a fetal monitor. Fetal monitors provide data regarding fetal movement and heart performance. Such monitors are particularly useful during the late stages of pregnancy. Fetal monitors are often used during the early stages of labor. Conventional fetal monitors include transducers placed on the maternal abdomen which transmit data via cables to a fetal monitoring station. Cables from these transducers require that the patient remain at or very close to the fetal monitor. The cables frequently tangle and are invariably in the way during monitoring.

One prior attempt to solve the problems associated with transducer cables includes the use of a low frequency magnetic or inductive coupling to couple signals from a transducer to a monitoring station. The signals are frequency modulated onto a low frequency carrier and output via a transducer coil to a receiver coil which receives the carrier by electromagnetic induction. The received signals are demodulated and processed by instrumentation coupled to the receiver coil. This approach, however, provides limited transmission distance, such as 2 or 3 feet. Further, if the coils are positioned at right angles to each other, there is no transmission.

Another attempted solution is an optical patient monitoring apparatus which uses an optical signal to transmit information from a transducer to a photodetector of an optical receiver. Physiological signals are modulated onto a carrier signal, and the resulting modulated signal is provided to an optical source (e.g., light-emitting diode or LED) which emits in the infrared band. This approach, however, provides limited battery life due to the high current drain of the LED's and has a limited range due to the light intensity. Further, a direct line of sight is required between the transducer and the photodetector, which can easily be obstructed by a patient, medical professional, medical instruments, etc.

Yet another attempt involves the use of radio transmission of digitalized and coded sensor data, for example, in the 1 Gigahertz range, from patient electrodes to an evaluator station. One drawback of radio transmission is the need for large antennas, which are sometimes positioned variously across the ceiling of a monitoring room. Furthermore, radio transmission requires more power than is practical in many applications.

Thus, there is a need for a wireless data communication system having an increased range from the transmit point to the receive point. There is further a need for a wireless data communication system having a decreased power dissipation. Also, there is a need for a wireless data communication system allowing easier movement of the patient without concerns for interruption in transmission. Even further, there is a need for a wireless data communication system in medical diagnostic systems which provides greater patient comfort and convenience.

One exemplary embodiment relates to a wireless data communication system including a transmit circuit configured to receive a data signal and to generate a high frequency sound signal based on the received data signal and a receive circuit spaced a distance from the transmit circuit configured to receive the high frequency sound signal and to remove the data signal from the high frequency sound signal.

Another exemplary embodiment relates to a transducer assembly for a patient monitoring system having a transducer, a transmit circuit, and a battery. The transducer is configured to receive a physiological signal from a patient and to generate a data signal representative of the physiological signal. The transmit circuit is coupled to the transducer and is configured to receive the data signal and to generate a high frequency sound signal based on the data signal. The battery is configured to provide power to the transducer and the communication circuit.

Yet another exemplary embodiment relates to a wireless data communication system having means for receiving a data signal, means for generating a high frequency sound signal based on the received signal at a first location, means for receiving the high frequency sound signal at a second location spaced a distance from the first location, and means for removing the data signal from the high frequency sound signal.

Other features and advantages of the present invention will become apparent to those skilled in the art upon review of the following drawings, the detailed description, and the appended claims.

The invention will now be described in greater detail, by way of example, with reference to the drawings, in which:-
FIGURE 1 is a diagram illustrating a wireless data communication system according to an exemplary embodiment of the invention;
FIGURE 2 is a block diagram of the wireless data communication system of FIG. 1; and
FIGURE 3 is a block diagram of the transmit circuit and the receive circuit of FIG. 2.

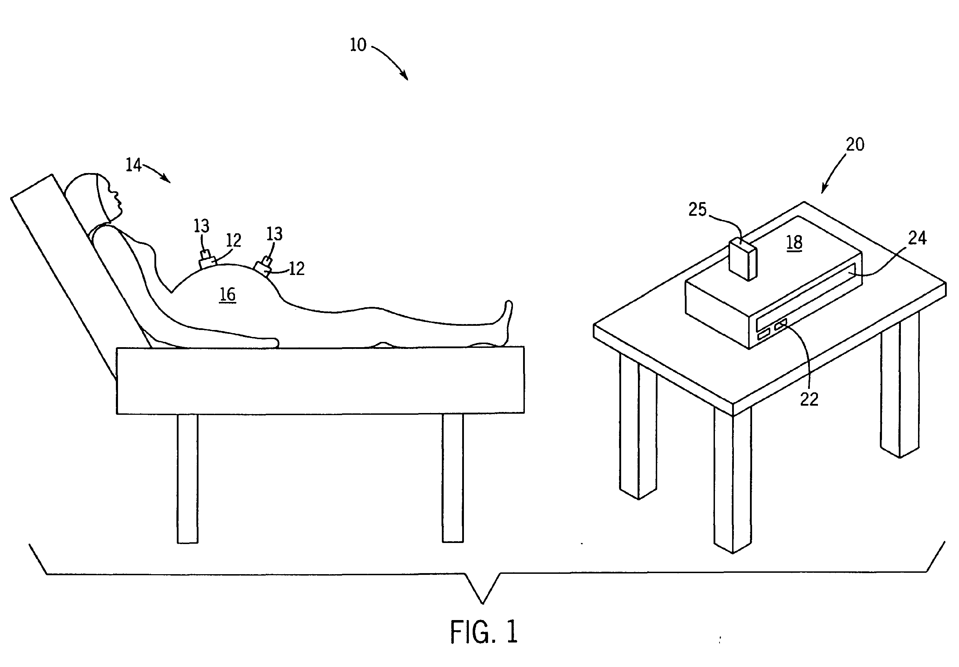

Referring first to FIG. 1, a wireless data communication system 10 according to an exemplary embodiment is shown. Wireless data communication system 10 is shown in a medical diagnostic implementation and, more specifically, in a fetal monitoring implementation. While many advantages of system 10 are realized in conjunction with a fetal monitoring system, system 10 also has advantages when implemented with other medical diagnostic applications and with data communication systems outside the medical field.

System 10 includes one or more transducer assemblies 12 located at a first location 14 (e.g., near a patient) configured to convert a physiological signal (e.g., signals indicating the functions or activities of organs, tissues, cells, etc., such as, fetal heart rate, uterine activity, etc.) from a patient 16 to a high frequency sound signal. Transducer assembly 12 includes a transducer, a transmit circuit having a transmit device 13 (e.g., a resonator, an electrostatic transceiver, an electromechanical transmitter, a piezoelectric device, a speaker, etc.), and a battery. High frequency sound, as used herein, refers to sound at or above the audible range of the human ear. The high frequency sound signal comprises a carrier signal at a carrier frequency and a data signal representative of the physiological signal. The high frequency sound signal is transmitted via transmit device 13 of transducer assembly 12 throughout the room in which patient 16 is located.

A monitoring station 18 is located at a second location 20 spaced a distance from first location 14. The distance may be as little as a few inches or less, or as great as one hundred feet or more, depending on the strength of the high frequency sound signal. Monitoring station 18 includes a user input device 22 (e.g., including buttons, switches, dials, a graphic user interface, a touch screen, etc.), an output device 24 (e.g., a display, a printer, a modem, etc.), and a receive device 25 (e.g., a resonator, an electrostatic transceiver, an electromechanical receiver, a piezoelectric device, a speaker, etc.). Receive device 25 receives the high frequency sound signal and provides the signal to monitoring station 18. Receive device 25 has the same structure as transmit device 13, but may have different structures in alternative embodiments.

Monitoring station 18 removes the data signal from the carrier signal and provides the data signal to the output device 24, for example, to a strip chart.

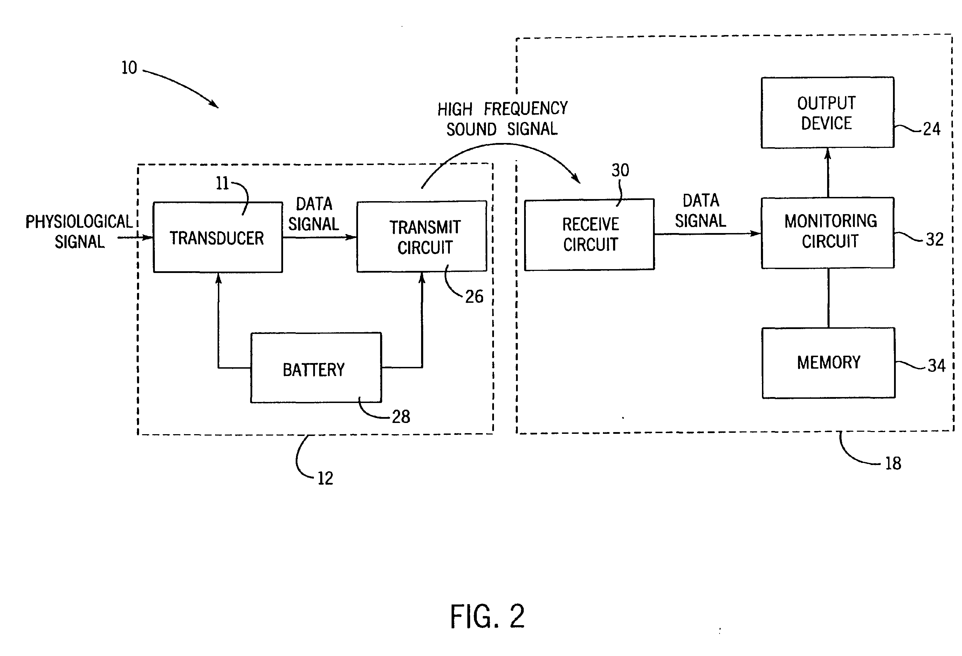

Referring now to FIG. 2, a block diagram of wireless communication system 10 is shown. Transducer assembly 12 includes a transducer 11 coupled to a transmit circuit 26. Transmit circuit 26 includes transmit device 13. Transducer 11 and transmit circuit 26 are powered by a battery 28. Battery 28 is, for example, an alkaline battery, a Nickel Cadmium battery, another rechargeable battery, etc. The greater the power available from battery 28, the higher the transmit frequency may be above the audible range. Transmit circuit 26 includes digital and/or analog circuitry.

In operation, transducer 11 senses a source signal (e.g., a physiological signal containing physiological data) and converts the source signal into an electrical data signal. Transducer 11 then provides the data signal to a transmit circuit 26 which includes digital and/or analog circuitry configured to generate a high frequency sound carrier signal at a carrier frequency and to modulate the data signal onto the carrier signal. Modulation can be by frequency modulation, amplitude modulation, phase modulation, etc. The carrier frequency is preferably between about 20 kilohertz (kHz) and 50 kHz, and also is preferably about 30 kHz. The higher the frequency, the greater are the losses in the signal. Transmit circuit 26 then provides the high frequency sound signal via transmit device 13 (see FIG. 1) to the surrounding environment.

Monitoring station 18 includes a receive circuit 30 (e.g., including oscillators, mixers, filters, etc.) coupled to a monitoring circuit 32 (e.g., including a microprocessor or other control circuitry configured to monitor, process, and synthesize data). Monitoring circuit 32 is further coupled to a memory 34 (e.g., random access memory, flash memory, hard disk storage, etc.) and an output device 24. Receive circuit 30 is configured to receive the high frequency sound signal via a receive device 25 (e.g., a resonator similar to transmit device 13). Receive circuit 30 is further configured to demodulate the data signal from the carrier signal and provide the data signal to the monitoring circuit 32. Monitoring circuit 32 stores data in a memory 34, processes the data, and provides selected data to output device 24. Monitoring circuit 32 may further be configured to process the data in a manner that assists a medical professional in diagnosing patient 16. In one embodiment, monitoring circuit 32 generates display signals and provides the display signals to a display such that the display provides viewable indicia representative of the data signal.

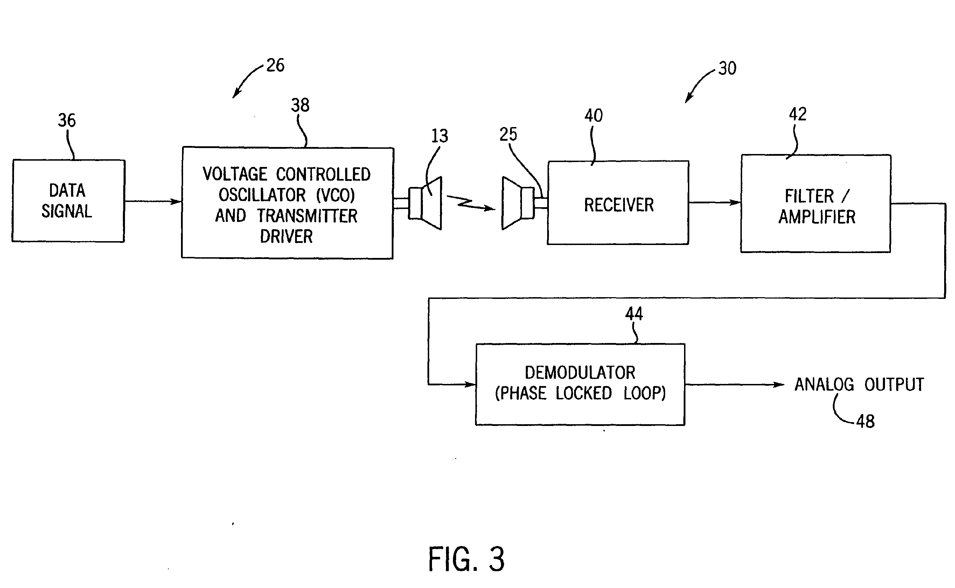

Referring now to FIG. 3, a more detailed block diagram of transmit circuit 26 and receive circuit 30 is shown. Data signal 36 from transducer 11 or another source is provided to a voltage controlled oscillator (VCO) and transmitter driver 38. A high frequency sound signal is generated by VCO and transmitter driver 38 and provided to transmit device 13 for transmission. Transmit device 13, in this exemplary embodiment, is a piezoelectric ceramic resonator designed to fill a room with high frequency sound. A suitable ceramic resonator includes a ceramic disk of approximately 1-inch diameter and a thickness of 0.035 inches. Transmit device 13 also requires very low power (e.g., on the order of milliWatts) to produce the high frequency sound signal. The larger the diameter of the ceramic disk, the lower the resulting frequency. The ceramic disk is mounted or clamped in a holder having a resonant cavity. The resonant cavity is configured to resonate at the carrier frequency.

Receive circuit 30 includes receive device 25 (e.g., a piezoelectric ceramic resonator similar to transmit device 13) configured to receive the high frequency sound signal and provide it to a receiver 40. Receiver 40 amplifies the received signal and provides it through a filter/amplifier 42 (e.g., including a lowpass filter, bandpass filter, and/or other amplifiers, filters, etc.) configured to remove the carrier frequency from the signal. Filter 42 then provides the filtered signal to a demodulator 44 such as a frequency demodulator, analog demodulator, etc., having a phase locked loop. Demodulator 44 provides an analog output signal 48 representative of the data signal. Receive circuit 30 may further include an additional stage to reduce multi-path or reverberation of the sound signal off of the walls of the room.

According to an alternative embodiment, transmit circuit 26 and receive circuit 30 may be configured for two-way and/or duplex communication such that monitoring station 18 can provide configuration data or other information to transducer assembly 12.

In summary, it is easier and more efficient to fill a room with sound than with light or magnetics. The exemplary embodiment presented herein provides one such embodiment of a wireless data communication system utilizing high frequency sound, but alternative configurations will become apparent to one of ordinary skill in the art utilizing the teachings presented herein.

While the embodiments illustrated in the FIGURES and described above are presently preferred, it should be understood that these embodiments are offered by way of example only. Other transmit and receive devices of various sizes and configurations may be substituted for the ceramic resonators presented herein. The data signal which is provided over the high frequency sound signal is presented herein as a physiological signal, but may alternatively be a digitized voice signal, electrocardiogram signal, financial information, internet data, etc. In one exemplary application, a series of patients could have transducer assemblies configured to monitor various vital signs, the transducer assemblies transmitting the data by high frequency sound to a nearby Internet or intranet station. The data would be converted from sound to electronic data and conveyed via the intranet station to a single location such that a patient caregiver can conveniently monitor a plurality of patients simultaneously.

For the sake of good order, various aspects of the invention are set out in the following clauses:-
1. A wireless data communication system, comprising:
   a transmit circuit configured to receive a data signal and to generate a high frequency sound signal based on the received data signal; and
   a receive circuit spaced a distance from the transmit circuit configured to receive the high frequency sound signal and to remove the data signal from the high frequency sound signal.
2. The wireless data communication system of clause 1, wherein the high frequency sound signal has a frequency between approximately 20 kilohertz and 50 kilohertz.
3. The wireless data communication system of clause 1, wherein the data signal represents physiological data.
4. The wireless data communication system of clause 3, further comprising a transducer configured to receive a physiological signal from a patient and to generate the data signal based on the physiological signal.
5. The wireless data communication system of clause 4, further comprising a battery configured to provide power to the transducer and the transmit circuit.
6. The wireless data communication system of clause 4, further comprising a monitoring circuit coupled to the receive circuit configured to record the data signal in a memory.
7. The wireless data communication system of clause 6, further comprising a display coupled to the monitoring circuit configured to provide a viewable indicia representative of the data signal.
8. The wireless data communication system of clause 1, wherein the transmit circuit includes a ceramic resonator configured to generate the high frequency sound signal.
9. The wireless data communication system of clause 1, wherein the transmit circuit is further configured to frequency modulate the data signal on a high frequency sound carrier signal to generate the high frequency sound signal.
10. A transducer assembly for a patient monitoring system, comprising:
   a transducer configured to receive a physiological signal from a patient and to generate a data signal representative of the physiological signal;
   a transmit circuit coupled to the transducer configured to receive the data signal and to generate a high frequency sound signal based on the data signal; and
   a battery configured to provide power to the transducer and the communication circuit.
11. The transducer assembly of clause 10, wherein the high frequency sound signal has a carrier frequency between approximately 20 kilohertz and 50 kilohertz.
12. The transducer assembly of clause 10, wherein the transmit circuit includes a ceramic resonator configured to generate the high frequency sound signal.
13. The transducer assembly of clause 10, wherein the transmit circuit is further configured to frequency modulate the data signal on a high frequency sound carrier signal.
14. A wireless data communication system, comprising:
   means for receiving a data signal;
   means for generating a high frequency sound signal based on the received signal at a first location;
   means for receiving the high frequency sound signal at a second location spaced a distance from the first location; and
   means for removing the data signal from the high frequency sound signal.
15. The wireless data communication system of clause 14, wherein the high frequency sound signal has a frequency between approximately 20 kilohertz and 50 kilohertz.
16. The wireless data communication system of clause 14, wherein the data signal represents physiological data.
17. The wireless data communication system of clause 16, further comprising a transducer means for receiving a physiological signal from a patient and for generating the data signal based on the physiological signal.
18. The wireless data communication system of clause 17, further comprising a means for providing power to the transducer means and the means for generating.
19. The wireless data communication system of clause 14, wherein the means for generating includes a ceramic resonator configured to generate the high frequency sound signal.
20. The wireless data communication system of clause 14, wherein the means for generating frequency modulates the data signal on a high frequency sound carrier signal.

## Claims

1. A wireless data communication system (10), comprising:
a transmit circuit (26) configured to receive a data signal and to generate a high frequency sound signal based on the received data signal; and
a receive circuit (30) spaced a distance from the transmit circuit (26) configured to receive the high frequency sound signal and to remove the data signal from the high frequency sound signal.

2. The wireless data communication system (10) of claim 1, wherein the high frequency sound signal has a frequency between approximately 20 kilohertz and 50 kilohertz.

3. The wireless data communication system (10) of claim 1, wherein the data signal represents physiological data.

4. The wireless data communication system (10) of claim 3, further comprising a transducer (11) configured to receive a physiological signal from a patient (16) and to generate the data signal based on the physiological signal.

5. A transducer assembly (12) for a patient monitoring system, comprising:
a transducer (11) configured to receive a physiological signal from a patient (16) and to generate a data signal representative of the physiological signal;
a transmit circuit (26) coupled to the transducer (11) configured to receive the data signal and to generate a high frequency sound signal based on the data signal; and
a battery (28) configured to provide power to the transducer (11) and the communication circuit (26).

6. The transducer assembly (12) of claim 5, wherein the high frequency sound signal has a carrier frequency between approximately 20 kilohertz and 50 kilohertz.

7. The transducer assembly (12) of claim 5, wherein the transmit circuit (26) includes a ceramic resonator configured to generate the high frequency sound signal.

8. A wireless data communication system (10), comprising:
means for receiving (30) a data signal;
means for generating (26) a high frequency sound signal based on the received signal at a first location (14);
means for receiving (30) the high frequency sound signal at a second location 20 spaced a distance from the first location (14); and
means for removing 32 the data signal from the high frequency sound signal.

9. The wireless data communication system (10) of claim 8, wherein the high frequency sound signal has a frequency between approximately 20 kilohertz and 50 kilohertz.

10. The wireless data communication system (10) of claim 14, wherein the data signal represents physiological data.
